# EUROPEAN PATENT APPLICATION

(11) **EP 2 087 898 A1**
(43) Date of publication of application: **12.08.2009**
(21) Application number: 08290106.7
(22) Date of filing: 06.02.2008
(51) Int. Cl.: A61K 35/74, A61K 39/04, A61P 37/00

(54) **A preparation of mycobacterium bovis BCG killed by extended freeze drying (EFD) for treating rheumatoid arthritis**

(71) Applicant: Institut Pasteur, 75015 Paris (FR); INSTITUT NATIONAL DE LA SANTE ET DE LA RECHERCHE MEDICALE (INSERM), 75013 Paris (FR)
(72) Inventor: Marchal, Gilles, 75014 Paris (FR); Lagranderie, Micheline, 92200 Neuilly sur Seine (FR); Boissier, Marie-Christophe, 75006 Paris (FR); Bessis, Natacha, 75011 Paris (FR)
(74) Representative: Leblois-Préhaud, Hélène Marthe Georgette

(57) **Abstract**

A preparation of *Mycobacterium bovis* BCG killed by Extended Freeze Drying (EFD) for treating rheumatoid arthritis.

## Description

The invention relates to a preparation of *Mycobacterium bovis* BCG killed by Extended Freeze Drying (EFD) for treating rheumatoid arthritis (RA).

Inflammation is a protective attempt by the organism to remove a harmful stimulus provoked by pathogens, physical harm, ischemic, toxic or autoimmune injury, as well as initiate the healing process for the tissue. Inflammation is a complex biological response of vascular tissues including the concerted and often opposing activities of several cell types and dozens of lipid and protein mediators.

Inflammation can be classified as either acute or chronic. Acute inflammation is a short-term process which is characterized by the classic signs of inflammation: swelling, redness, pain, heat, and loss of function. It occurs as long as the injurious stimulus is present and ceases once the stimulus has been removed, broken down, or walled off by scarring. Acute inflammation is the initial response of the body to harmful stimuli and is achieved by the increased movement of plasma proteins and circulating cells (neutrophils, dendritic cells, monocytes, mast cells and lymphocytes) from the blood into the injured tissues. A cascade of biochemical events propagates and matures the inflammatory response, involving the local vascular system, the immune system, and various cells within the injured tissue. Finally, down-regulation of the inflammatory response concludes acute inflammation. Removal of the injurious stimuli halts the response of the inflammatory mechanisms, which require constant stimulation to propagate the process. Additionally, many inflammatory mediators have short half lives and are quickly degraded in the tissue, helping to quickly cease the inflammatory response once the stimulus has been removed.

However, if the delicate balance between inflammation and restoration is broken, it leads to chronic inflammation and to the development of chronic inflammatory diseases such as rheumatoid arthritis.

Chronic inflammation, leads to a progressive shift in the cells which are present at the site of inflammation to mononuclear immune cells (macrophages, lymphocytes and mast cells) and is characterized by simultaneous tissue destruction and attempts at repair (angiogenesis and fibrosis) of the tissue from the inflammatory process. It may follow active inflammation, but more often it starts as a low-grade response and causes tissue damage over a long period of time such as arthritis. Rheumatoid arthritis (RA) is a chronic systemic inflammatory disease mostly known as a polyarticular joint disease characterized by synovial proliferation in the joint, infiltration of the synovial stroma by B cells, CD4+ helper cells, plasma cells and macrophages. Other histological features include hypervascularisation, increased osteoclast activity and pannus formation consisting of a mass of synovium, inflammatory cells and fibroblasts causing destruction and ossification of the adjacent cartilage and bone. Mouse collagen-induced arthritis is a typical model for rheumatoid arthritis (Miellot et al., Eur. J. Immunol., 2005, 35, 3704-3713).

The pathogenesis of rheumatoid arthritis is still not clear and depends at least in part on cytokine dysregulation within altered tissue (TNF-α, IL-6, IL-1β, IL-17, IFN-γ, IL-12p40, MIG). In particular, the proinflammatory cytokine TNFα was shown to be critically involved in the pathogenesis of RA (Le Buanec et al., Proc. Natl. Acad. Sci. USA, 2006, 13, 19442-19447). Several studies suggest that the susceptibility to chronic inflammatory diseases is associated with dysfunctions of regulatory T lymphocytes (Miellot et al. Eur. J. Immunol., 2005, 35, 3704-3713) and/or, either a Th1-dominant (TNF-α, IFN-γ, IL-12, IL-6 IL-1β) or a Th2-dominant (IL-4, IL-13, IL-5 ) immune response (Th1/Th2 imbalance).

NF-κB/Rel transcription family including the most prevalent activated form of NF-κB (heterodimer p50/p65 or p52/p65) is highly activated at sites of inflammation in diverse chronic inflammatory diseases and can induce transcription of proinflammatory cytokines, chemokines, adhesion molecules, MMPs, Cox-2, and inducible nitric oxide (Tak et al., The Journal of Clinical Investigation, 2001, 107, 7-11). Peroxisome proliferator activated receptor γ (PPARγ) is a ligand activated transcription factor belonging to the nuclear hormone receptor superfamily that is expressed in various immune cells. Activators of PPARγ, including natural (fatty acids, oxidized fatty acids, eicosanoids) and synthetic (hypolipidemic drugs (fibrates), the antidiabetic thiazolidinediones (TZD) and certain nonsteroidal anti-inflammatory drugs (NSAID)) products, inhibit the expression of proinflammatory genes by antagonizing NF-κB, AP-1, NF-AT and STAT signaling pathways (Fahmi et al., The Journal of Rheumatology, 2002, 29, 1-14). The role of PPARγ in the control of the inflammatory response was confirmed in models of chronic inflammatory processes including rheumatoid arthritis, and also in clinical studies (Szèles et al., Biochemica et Biophysica Acta, 2007, 1771, 1014-1030).

Inflammatory syndromes are chronic pathologies which are disabling and which may be mortal. The medical treatment for these inflammatory syndromes is essentially based on the use of powerful anti-inflammatory agents, including non-steroïd drugs (NSAIDs) and glucocorticoid drugs, antimitotics, and more recently TNFα blockers (Le Buanec et al., Proc. Natl. Acad. Sci. USA, 2006, 13, 19442-19447).

Non-Steroid Anti-Inflammatory Drugs (NSAIDs) such as aspirin and ibuprofen, have analgesic, antipyretic and anti-inflammatory properties. The NSAIDs are inhibitors of the cyclo-oxygenase isoforms COX-1 and COX-2, two key enzymes in prostaglandin, thromboxan and other eicosanoïds synthesis from arachidonic acid. The expression "non-steroid" or "non-steroidal" is used to differentiate the NSAIDs from the glucocorticoids drugs, which among a large panel of effects, have a similar anti-inflammatory effect (depression of eicosanoids). NSAIDs have well-known digestive (nausea, ulcer, diarrhea) and renal side-effects which are relatively frequent. These side effects account for 20 % of the hospitalizations for cardiac insufficiency. Rofecoxib (COX-2 inhibitor) which is responsible for heart-attacks probably by increasing plattelets aggregation, has been withdrawn from market. Therefore, the search for inhibitors of COX-3 whose specificity is not as clear as that of COX-1 and COX-2 is in progress.

Glucocorticoids are the other main class of anti-inflammatory drugs used for treating inflammatory syndromes since the 1950s. The anti-inflammatory effect of glucocorticoids is secondary to an increase in lipocortin 1 synthesis, whose binding to cell membranes inhibits the phospholipase A2 action. The prostaglandin synthesis is reduced, all the more so that there is a combined inhibition of cyclo-oxygenases. The targets of glucocorticoids are thus partly upstream of those of NSAIDs. The high efficacy of glucocorticoïds is however accompanied with marked side-effects. Hypokaliemia, hyponatremia, hypertension and Cushing syndromes occur after prolonged systemic treatment with glucocorticoid. Bone demineralisation and pheripheral oedema are known also, but rare, due to the arrest or the diminution of the treatment which is still needed for chronic inflammatory diseases like RA. Local treatments, as those used for asthma induce little side-effects. Nevertheless, of note is the recent report of an association between local treatment with glucocorticoids (nasal sprays, dermic cream) and central serous retinopathy which can result in major visual troubles.

Today, several anti-TNFα monoclonal antibodies (infliximab, adalimumab) and a soluble hTNFα receptor derivative (etanercept), specifically targeting hTNF α are used in therapy of autoimmune diseases. However, potential side effects of the TNFα blockers include the risk of infection (sepsis or tuberculosis), the occurrence of anti-DNA autoAbs and possibly lymphoma. Active anti-TNFα immunization with a biologically inactive but immunogenic hTNFα derivative (TNFα kinoid) would limit the rate of therapeutic failure of anti-TNFα monoclonal antibodies due to the generation of antiidiotypic antibodies. However, the TNFα kinoid which inhibits the TNFα component of the Th1 immune response does not prevent the risk of infection associated with the use TNFα blockers.

Any treatment bringing about a decrease in the doses of anti-inflammatory agents to be administered or that may be substituted to these anti-inflammatory agents are important.

*Mycobacteria bovis* BCG killed by Extended Freeze Drying (EFD) has potent anti-inflammatory activities in several animal models of allergy (asthma; International PCT Application WO 03/049752) and intestinal inflammatory disease (inflammatory bowel disease; International PCT Application WO 2007/072230). EFD has the original property of acting not only on the Th1 but also on the Th2 signalling pathway (International PCT Application WO 2007/072230). The protective/curative effect of EFD against the symptoms of the disease is associated with CD4+ CD25+ regulatory cells producing IL-10 (International PCT Application WO 03/049752). Moreover EFD treatment does not induce toxic effects or delayed-type hypersensitivity and side effects associated with live or heat-killed BCG.

By using mice experimental models, the inventors have demonstrated that EFD can treat rheumatoid arthritis. The injection of EFD at the onset of the clinical signs of arthritis reduces significantly the clinical scores of inflammation by the induction of an immunoregulatory response (expression of FOXP-3 and IL-10 production). In addition, EFD treatment increased T-bet, signature of Th1 response. Therefore, EFD represents a new alternative to TNFα blockers or soluble TNFα that would have the advantage of not inhibiting the Th1 immune response, thereby limiting the risk of infection (sepsis or tuberculosis).

The invention relates to a preparation of *Mycobacterium bovis* BCG killed by extended freeze drying (EFD) for treating rheumatoid arthritis.

The expression "extended freeze-dried *Mycobacterium bovis* BCG preparation", "EFD preparation" or "EFD" as used in the context of the present invention refers to killed *Mycobacterium bovis* BCG bacteria prepared according to the method as described in the International PCT Application WO 03/049752, said method comprising the steps of: (i) harvesting a culture of live bacteria cells, (ii) washing the bacteria cells in water or in an aqueous solution of a salt, (iii) freezing the bacteria cells in water or in an aqueous solution of a salt, (iv) killing the frozen bacteria cells by drying them in a lyophiliser, for a time sufficient to remove at least 98.5% of the water, preferably at least 99 % of the water, more preferably at least 99.5 % of the water, and (v) collecting the extended freeze-dried killed bacteria cells.

The expression "EFD" as used in the context of the present invention refers in other words to killed *Mycobacterium bovis* BCG bacteria where the structure of the molecules and in particular the structure of the proteins is preserved. An example of preserved protein is Apa which has the same migrating characteristics in a SDS-PAGE gel as the protein extracted from living mycobacteria (Laqueyrerie et al., Infect. Immun., 1995, 63, 4003-4010).

A fraction of EFD preparation as described above is covered by the expression "EFD" or "EFD preparation" of the invention. This fraction is selected in the group consisting of: a fraction consisting of an organic solvent extract of said EFD preparation, a fraction consisting of a glycosidase-treated extract of said EFD preparation, a fraction consisting of a DNase and/or a RNase-digested extract of said EFD preparation, a fraction consisting of a protease-treated extract of said EFD preparation, a fraction consisting of said EFD preparation successively treated by an organic solvent, a glycosidase, a DNase and/or a RNase, and finally a protease, and a fraction consisting of said EFD preparation treated by a protease (as substilisine for example) and a DNase.

According to another advantageous embodiment of the invention, said preparation is included in a pharmaceutical composition additionally comprising an acceptable carrier and/or an additive and/or an immunostimulant and/or an adjuvant.

Any suitable carrier known to those of ordinary skill in the art may be employed in the pharmaceutical composition of the present invention, the type of carrier varying depending on the mode of administration. For parenteral administration, such as subcutaneous injection, the carrier preferably comprises water, saline buffer, lactose, mannitol, glutamate, a fat or a wax and the injectable pharmaceutical composition is preferably an isotonic solution (around 300-320 mosmoles). For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (e.g. polylactic galactide) may also be employed as carriers for the pharmaceutical compositions of this invention. Suitable biodegradable microspheres are disclosed, for example in US Patent 4,897,268 and 5,075,109. The additive may be chosen among antiaggregating agents, antioxidants, dyes, flavor enhancers, or smoothing, assembling or isolating agents, and in general among any excipient conventionally used in the pharmaceutical industry. Any of the variety of adjuvants/immunostimulants may be employed in the compositions of the present invention to enhance the immune response. Most adjuvants contain a substance designed to protect the antigen from rapid catabolism or to create controlled inflammatory reactions, such as aluminium hydroxide or mineral oil, and a non-specific stimulator of immune response, such as lipid A, *Bordetella pertussis* toxin. Suitable adjuvants are commercially available as, for example, Freund's Incomplete Adjuvant and Freund's complete adjuvant which can not be used for injection in human. Other suitable adjuvants which can be used in human include aluminium hydroxide, biodegradable microspheres, monophosphoryl A and Quil A.

The pharmaceutical composition may be in a form suitable for oral administration. For example, the composition is in the form of tablets, ordinary capsules, gelatine capsules or syrup for oral administration. These gelatine capsules, ordinary capsules and tablet forms can contain excipients conventionally used in pharmaceutical formulation, such as adjuvants or binders like starches, gums and gelatine, adjuvants like calcium phosphate, disintegrating agents like cornstarch or algenic acids, a lubricant like magnesium stearate, sweeteners or flavourings. Solutions or suspensions can be prepared in aqueous or non-aqueous media by the addition of pharmacologically compatible solvents. These include glycols, polyglycols, propylene glycols, polyglycol ether, DMSO and ethanol.

In general, the composition may be administered by parenteral injection (e.g., intradermal, intramuscular, intravenous or subcutaneous), intranasally (e.g. by aspiration or nebulization), orally, sublingually, or topically, through the skin or through the rectum.

The amount of EFD preparation present in the composition of the present invention is a therapeutically effective amount. A therapeutically effective amount of EFD preparation is that amount necessary so that the EFD preparation performs its role of inhibiting the symptoms of inflammation without causing, overly negative effects in the subject to which the composition is administered. The exact amount of EFD preparation to be used and the composition to be administered will vary according to factors such as the type of inflammatory disease and the species (human, animal) being treated, the mode of administration, the frequency of administration as well as the other ingredients in the composition.

Preferably, the composition is composed of from about 10 µg to about 10 mg and more preferably from about 100 µg to about 1 mg, of EFD preparation. By "about", it is meant that the value of said quantity (µg or mg) of EFD preparation can vary within a certain range depending on the margin of error of the method used to evaluate such quantity.

For instance, during an oral administration of the composition of the invention, individual to be treated could be subjected to a 1 dose schedule of from about 10 µg to about 10 mg of EFD preparation per day during 3 consecutive days. The treatment may be repeated once one week later.

For parenteral administration, such as subcutaneous injection, the individual to be treated could be subjected to a 1 dose schedule of from about 10 µg to about 10 mg and more preferably from about 100 µg to about 1 mg, of EFD preparation per month or every 6 months.

The invention also relates to products containing an EFD preparation as defined in the invention or fractions thereof and a second product which is different from the first one, said second product being selected from the group consisting of anti-inflammatory and immunomodulatory drugs, as a combined preparation for simultaneous, separate or sequential use in the prevention and/or the treatment of rheumatoid arthritis.

The present invention will be further illustrated by the additional description and drawings which follows, which refers to examples illustrating the protective effect and the immune response induced by EFD in an experimental model of arthritis in mice. It should be understood however that these examples are given only by way of illustration of the invention and do not constitute in anyway a limitation thereof.

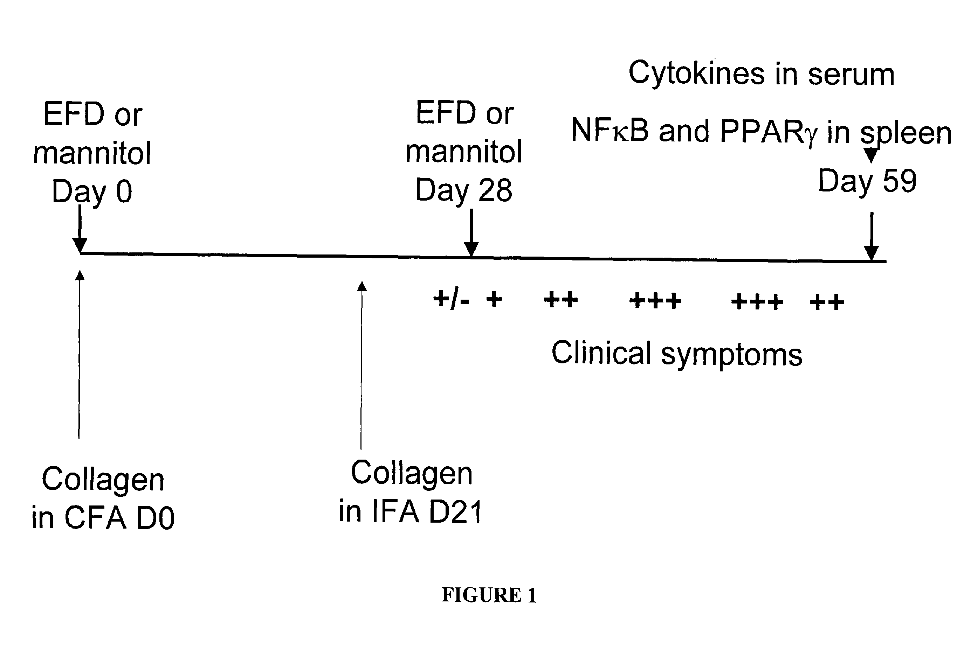

- figure 1 represents the experimental protocol for evaluating the effect of EFD treatment in the mouse model of collagen induced arthritis. Male DBA/1 mice aged 5-7 weeks were injected (Day 0) with native bovine type II collagen (CII) in complete Freund adjuvant. On day 21, mice were boosted with a subcutaneous injection of CII in incomplete Freund adjuvant. Mice were treated with EFD (100 µg, subcutaneous injection), either on Day 0 (early treatment) or on Day 28, at the onset of the first clinical signs (late treatment). Controls were treated with isotonic mannitol (5 %) in the same conditions. Clinical severity of arthritis was scored twice a week until the end of the experiment (Day 59). On Day 59, spleen, sera and hind legs were collected for further studies. Local (popliteal lymph nodes) and systemic (spleen and serum) immune responses were evaluated by measuring: (i) NFκB activation which in turn activates pro-inflammatory cytokines genes, (ii) PPAR-γ, a NFκBp65 antagonist, (iii) transcription factors which are signatures of Th1 (T-bet), Th2 (GATA-3) and T regulatory (FOXP3) responses, (iv) pro and anti-inflammatory cytokines.
- figure 2 represents the clinical scores of arthritis in mice treated with EFD at day 0 (-△-; early treatment) or day 28 (-∇-; late treatment) by comparison with controls (-●-). Statistical difference with control are indicated: * : p ≤ 0.05 **: p ≤ 0.01 ***: p ≤ 0.001.
- figure 3 illustrates the reduction of NFκBp65 activation in spleen nuclear extracts of mice treated with EFD at day 0 or day 28 by comparison with that of controls (mannitol). Statistical difference with control are indicated: * : p ≤ 0.05 ***: p ≤ 0.001.
- figure 4 illustrates the increase of PPAR-γ in spleen nuclear extracts of mice treated with EFD at day 0 or day 28 by comparison with that of controls (mannitol). Statistical difference with control are indicated: ** : p ≤ 0.01.
- figure 5 illustrates the effect of EFD treatments (early and late) on the expression of GATA-3, signature of Th2 immune response and T-bet and FOXP3, signatures of Th1 and Treg responses, respectively. Stat 1 is a factor of signalling and transcription signature of Th2 immune response.

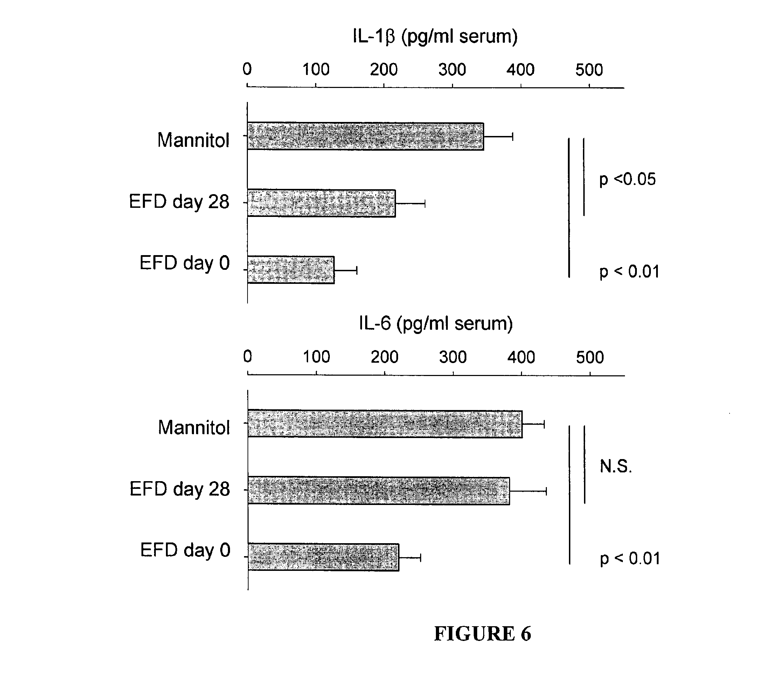

- figure 6 illustrates the effect of EFD treatments (early and late) on IL-1β and IL-6 levels in sera. A statistical difference with control is indicated by the p value. N.S.: non-significant difference.

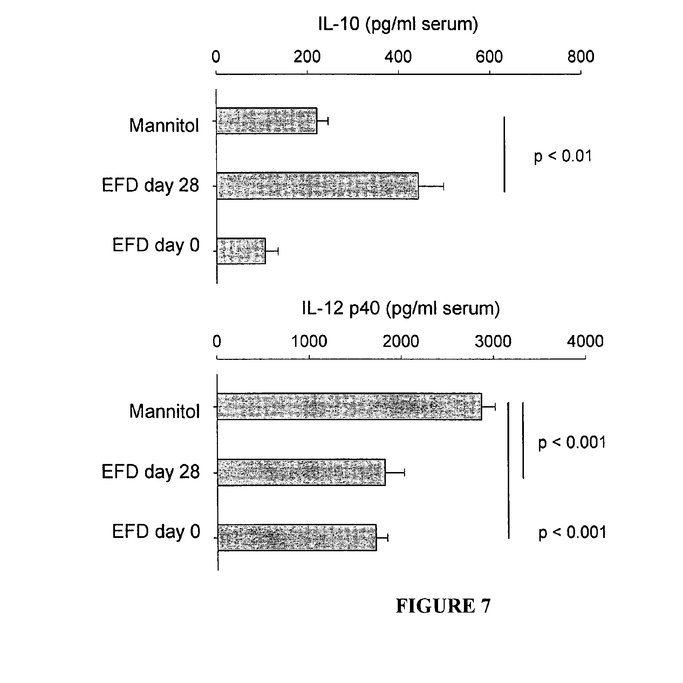

- figure 7 illustrates the effect of EFD treatments (early and late) on IL-10 and IL-12p40 levels in sera. A statistical difference with control is indicated by the p value. N.S.: non-significant difference.

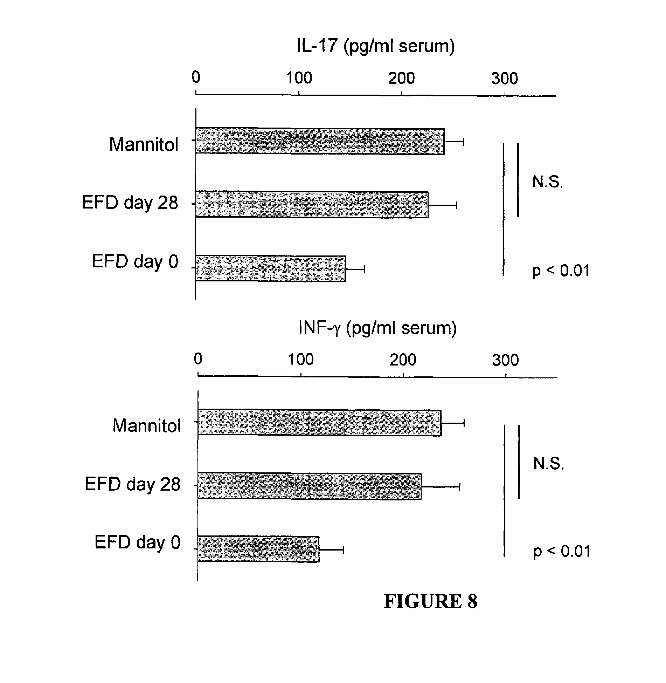

- figure 8 illustrates the effect of EFD treatments (early and late) on IL-17 and IFN-γ levels in sera. A statistical difference with control is indicated by the p value. N.S.: non-significant difference.

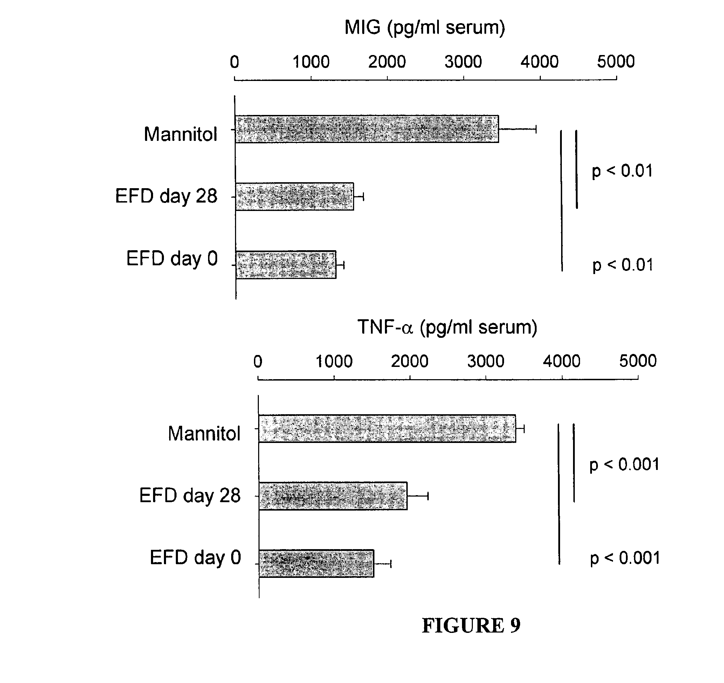

- figure 9 illustrates the effect of EFD treatments (early and late) on MIG and TNF-α levels in sera. A statistical difference with control is indicated by the p value. N.S.: non-significant difference.

### EXAMPLE 1: Preparation of EFD

*Mycobacterium bovis* BCG Pasteur strain (1173P2) killed by extended freeze-drying (EFD) was prepared as described previously in the International PCT Application WO 03/049752. The EFD preparation contained less than 0.5 % water at the end of the procedure, as determined with a coulometer by using the Karl-Fischer method (METROHM). Ten milligrams of EFD was cultured on Middlebrook 7H10 agar plates to confirm the absence of living bacteria.

The EFD was resuspended in mannitol (5 %) at a final concentration of 1 mg/mL, freeze-dried for 48 hours following the standard method for preparation of live BCG vaccine (see figure 1A of International PCT Application WO 03/049752) and finally dissolved in distillated water (final concentration of 1 mg/ml) before to be injected to mice.

### EXAMPLE 2: Evaluation of EFD treatment in the mouse model of collagen induced arthritis (CIA)

### 1) Material and methods

### a) Experimental protocol of arthritis induction/treatment in mice

Arthritis was induced with native bovine type II collagen (CIIb, (Chondrex, MORWELL DIAGNOSTICS) as previously described (Courtenay et al., Nature, 1980, 283, 666-668 ; Saidenberg-Kermanacc'h et al., J. Clin. Immunol., 2004, 24, 370-378 ; Miellot et al., Eur. J. Immunol., 2005, 35, 3704-3713).

Male DBA/1 mice (HARLAN) aged 5-7 weeks were distributed in three groups of mice (n=8 for each group; Figure 1) :
- **Grouρ A :** early EFD treatment : On day 0, mice were injected subcutaneously at the base of the tail with 100 µg CIIb (25 µL) emulsified in 25 µL complete Freund adjuvant (CFA, SIGMA-ALDRICH). On the same day (Day 0), they were injected subcutaneously at the base of the tail with 100 µg EFD (1 mg/ml) prepared as described above in example 1. On day 21, mice were boosted with a subcutaneous injection of CIIb (100 µg/25 µL) in 25 µL incomplete Freund adjuvant (IFA ; DIFCO LABORATORIES).
- **Groun B :** late EFD treatment ; On day 0, mice were injected subcutaneously at the base of the tail with 100 µg CIIb (25 µL) emulsified in 25 µL complete Freund adjuvant (CFA, SIGMA-ALDRICH). On day 21, mice were boosted with a subcutaneous injection of CIIb (100 µg/25 µL) in 25 µL incomplete Freund adjuvant (IFA ; DIFCO LABORATORIES). On day 28, at the onset of the first clinical signs of arthritis, mice were injected subcutaneously at the base of the tail with 100 µg EFD (1 mg/ml) prepared as described above in example 1.
- **Group C :** control ; On day 0, mice were injected subcutaneously at the base of the tail with 100 µg CIbI (25 µL) emulsified in 25 µl complete Freund Adjuvant (CFA, SIGMA-ALDRICH). On the same day, they were injected subcutaneously with 100 µL of a 5 % mannitol solution. On day 21, mice were boosted with a subcutaneous injection of CIIb (100 µg/25 µL) in 25 µL incomplete Freund adjuvant (IFA ; DIFCO LABORATORIES).

### b) Clinical evaluation of arthritis

Mice were monitored for evidence of arthritis twice a week until the end of the experiment (Day 59).

A blind procedure was used to monitor the mice for evidence of arthritis (Boissier et al., Arthritis Rheum., 1990, 33, 1-8). Briefly, clinical severity of arthritis in each joint or group of joints (toes, tarsus, and ankles) was scored by the same observer as follows: 0, normal; 1, erythema; 2, swelling; 3, deformity; and 4, necrosis. These scores were totaled to obtain the clinical arthritis score; the mean clinical arthritis score in each group was used to evaluate CIA severity on each clinical observation day. In addition, the maximal arthritis score (which is the mean of the maximal arthritis score of each mouse in each group) was used to evaluate disease severity over the study period. The mean arthritic score on each clinical observation day was calculated in each treatment group. All statistics were down using the Graph pad Instat Software. For all results a Mann-Whitney test was used. Differences were considered significant when p < 0.05.

### c) ELISA assays

NFκBp65 active form and PPAR-γ levels in spleen nuclear extracts, were measured using TransAM™ transcription factor assay kits (Active motif USA) according to the manufacturer's recommendations. Cytokines levels in serum were measured using Bioplex method according to the manufacturer's instructions (BIO-RAD).

### d) Immunoblotting

Total proteins extracted from the spleen cells were resolved on 7.5% SDS-PAGE, then protein bands transferred to nitrocellulose sheets were probed with polyclonal rabbit anti-mouse FOXP3 IgG kindly provided by E. Schmitt and C. Richter (Institute of Immunology, Mainz, Germany) with mouse monoclonal anti-T-bet, mouse monoclonal anti-GATA-3 (SANTA CRUZ BIOTECHNOLOGY), β-actin mouse monoclonal Ab (Ac-15; ABCAM), with anti-Stat 1 (SANTA CRUZ BIOTECHNOLOGY). HRP-labeled polyclonal goat anti rabbit (DAKO CYTOMATION) was used as secondary Ab. The immune complexes were revealed by enhanced chemiluminescence detection system (AMERSHAM,).

### 2) Results

### a) Clinical evaluation of arthritis in EFD treated mice

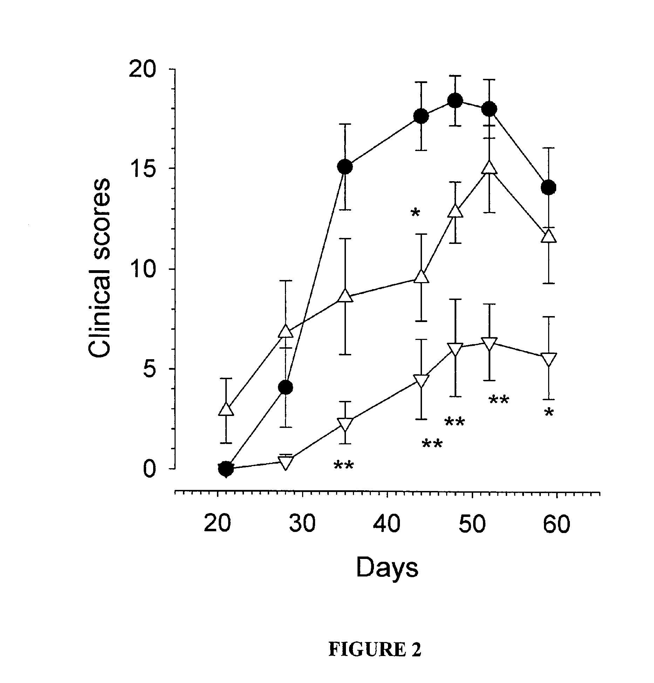

The control mice presented an increasingly elevated score of arthritis from day 28 (onset of clinical signs). Mice treated with EFD at day 28 (late treatment) presented a significantly reduced clinical score of arthritis (erythema, swelling, deformity and necrosis) from the seventh day of EFD treatment to the end of the experiment (Day 35 to Day 59; figure 2). By contrast, no significant effect was observed when EFD was administered before the clinical signs of arthritis (preventive treatment).

### b) Analysis of the immune response

Local (popliteal lymph nodes) and systemic (spleen and serum) immune responses after EFD late and early treatments were evaluated at the end of the experiment (day 59), by measuring: (i) NFκB activation (NFκB p65 active form) which in turn activates pro-inflammatory cytokines genes, (ii) PPAR-γ, a NFκBp65 antagonist, (iii) transcription factors which are signatures of Th1 (T-bet), Th2 (GATA-3, Stat 1) and T regulatory (FOXP3) responses, (iv) pro and anti-inflammatory cytokines.

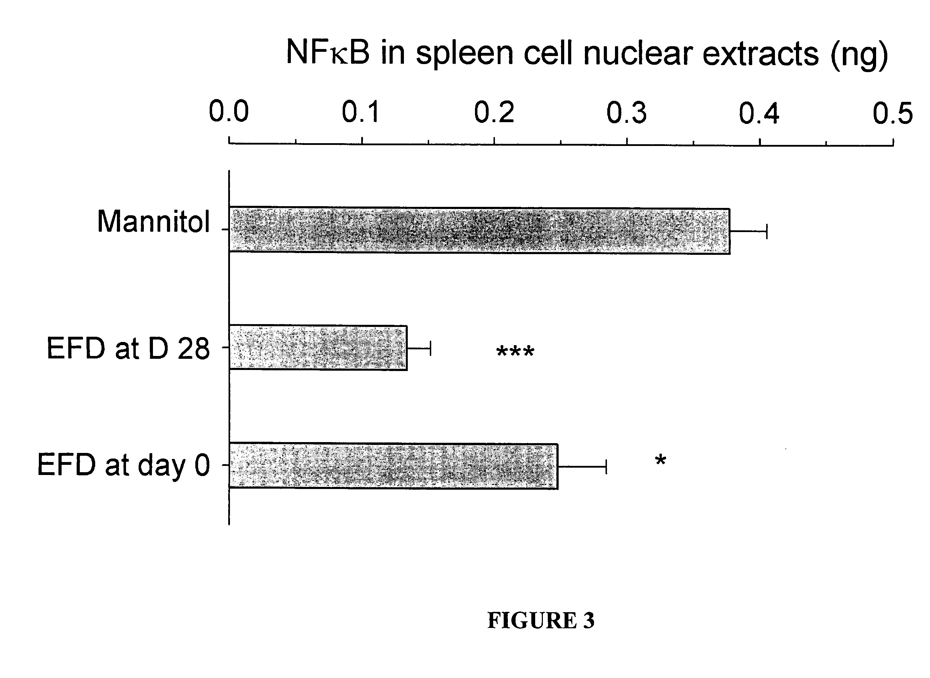

A strong reduction of NFκB activation was observed in spleen cell nuclear extracts of mice treated with EFD at day 28 (late treatment), whereas a slight reduction only was observed in mice treated with EFD at day 0 (Figure 3).

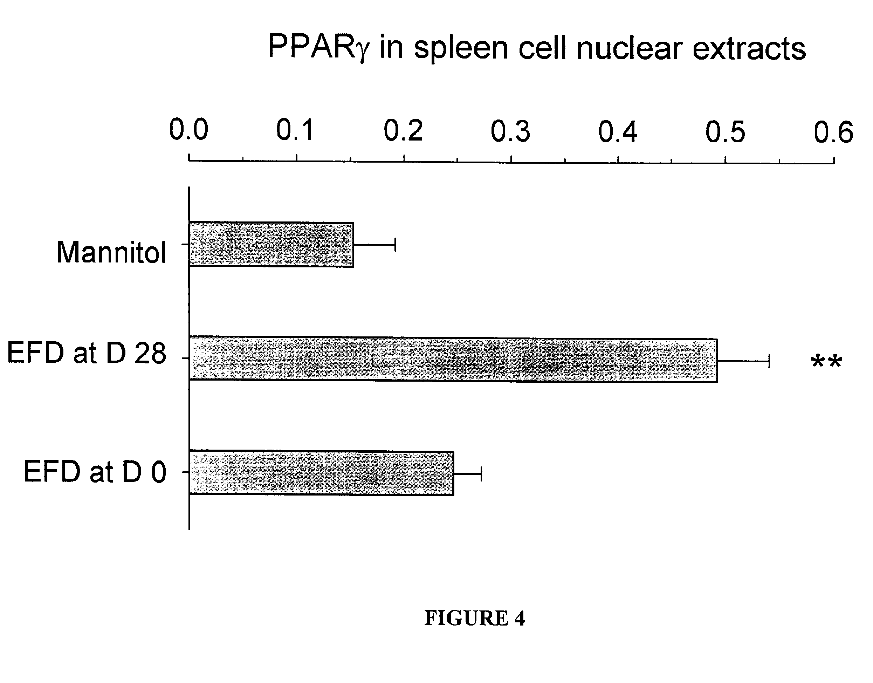

A strong increase of PPAR-γ level was observed in spleen cell nuclear extracts of mice treated with EFD at day 28 (late treatment). By contrast, PPAR-γ level in mice treated with EFD at day 0 showed no statistical differences with that of the control mice (Figure 4).

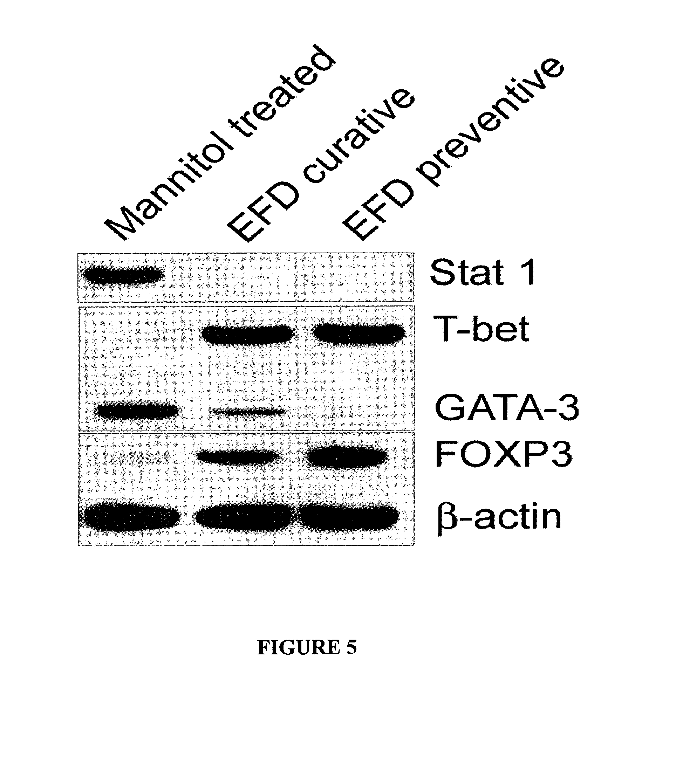

EFD treatments (early and late) reduced GATA-3 and Stat 1, signature of Th2 immune response and increase T-bet and FOXP3, signatures of Th1 and Treg responses, respectively (figure 5).

Cytokines involved in inflammatory processes were significantly reduced in sera, after EFD late and early treatments (IL-1β, TNF-α, IL-12p40, MIG) or only after the early treatment (IL-6, IFN-γ, IL-17). By contrast, late EFD treatment only induced a significant increase of the immunoregulatory cytokine IL-10. Other cytokines were also measured in serum at day 59, no modification was observed for: IL-4, IL-12p70, IL-13, MCP-1, MIP-1α, MIP-1β, RANTES, IL615, IL-18 and MIP-2.

The injection of EFD at the onset of the clinical signs of arthritis reduces significantly the clinical scores of inflammation by the induction of an immunoregulatory response (expression of FOXP-3 and IL-10 production).

## Claims

1. A preparation of *Mycobacterium bovis* BCG killed by extended freeze drying for treating rheumatoid arthritis.

2. The preparation according to claim 1, wherein the structure of the *Mycobacterium bovis* BCG proteins is preserved.

3. The preparation according to claim 1 or claim 2, which is composed of from about 10 µg to about 10 mg of *Mycobacterium bovis* BCG killed by extended freeze drying.

4. The preparation according to claim 3, which is composed of from about 100 µg to about 1 mg of *Mycobacterium bovis* BCG killed by extended freeze drying.

5. The preparation according to anyone of claims 1 to 4, which is obtainable by a process comprising:
(i) harvesting a culture of live *Mycobacterium bovis* BCG bacteria cells,
(ii) washing the *Mycobacterium bovis* BCG bacteria cells in water or in an aqueous solution of a salt,
(iii) freezing the *Mycobacterium bovis* BCG bacteria cells in water or in an aqueous solution of a salt,
(iv) killing the frozen *Mycobacterium bovis* BCG bacteria cells by drying them in a lyophiliser, for a time sufficient to remove at least 98.5% of the water, and
(v) collecting the extended freeze-dried *Mycobacterium bovis* BCG killed bacteria cells.

6. Products containing a preparation as defined in anyone of claims 1 to 5 and at least another product selected from the group consisting of anti-inflammatory and immunomodulatory drugs, as a combined preparation for simultaneous, separate or sequential use in the prevention and/or the treatment of rheumatoid arthritis.
